# EUROPEAN PATENT APPLICATION

(11) **EP 3 616 757 A1**
(43) Date of publication of application: **04.03.2020**
(21) Application number: 19194773.8
(22) Date of filing: 30.08.2019
(51) Int. Cl.: A61Q 19/00, A61K 8/9728, A61K 8/9789, A61K 8/34

(54) **TOPICAL COMPOSITIONS COMPRISING PICHIA ANOMALA AND A SOY PRODUCT**

(30) Priority: 30.08.2018 US 201862724807 P
(71) Applicant: JOHNSON & JOHNSON CONSUMER INC., Skillman, NJ 08558 (US)
(72) Inventor: LIU-WALSH, Fang, Skillman, NJ 08558 (US); GARAY, Michelle, Pittstown, NJ 08867 (US); MAITRA, Prithwiraj, Hillsborough, NJ 08844 (US); RANDHAWA, Manpreet, Robbinsville, NJ 08691 (US)
(74) Representative: Kirsch, Susan Edith

(57) **Abstract**

The present invention provides a topical composition comprising an extract of *Pichia anomala* and a soy product.

## Description

### CROSS-REFERENCE TO RELATED APPLICATIONS

This application claims the benefit of US provisional application 62/724807 filed on August 30, 2018, the complete disclosure of which is hereby incorporated herein by reference for all purposes.

### FIELD OF THE INVENTION

The present invention provides a method of treating skin by topically applying to skin a combination of an extract of *Pichia anomala* and a soy product. Additionally, a topical composition comprising a combination of an extract of *Pichia anomala* and a soy product is provided.

### BACKGROUND OF THE INVENTION

Hyaluronic acid is found in skin at the periphery of collagen and elastin fibers and where these fibers intersect. Hyaluronic acid is localized not only in the dermis but also in the epidermal intercellular spaces, especially the middle spinous layer, but not in the stratum corneum (SC) or stratum granulosum. In aged skin, the level of hyaluronic acid decreases and it disassociates from collagen and elastin. Skin containing reduced levels of hyaluronic acid also demonstrates reduced water binding, which may be involved in the changes noted in aged skin, including wrinkling, altered elasticity, reduced turgidity and diminished capacity to support the microvasculature of the skin. As one of the primary GAGs, hyaluronic acid can bind 1000 times its weight in water, and may help the skin retain and maintain water. It is found in all connective tissue and is produced mainly by fibroblasts and keratinocytes in the skin.

Different methods have been proposed for combating wrinkles and fine lines, including injection of hyaluronic acid. Injection of exogenous hyaluronic acid is used as a temporary dermal filling agent in soft tissue augmentation procedures. However, injected hyaluronic acid has a limited lifetime. On the other hand, penetration of exogenous hyaluronic acid into the skin has proved difficult to accomplish by topical application.

*Pichia* is a genus of yeasts in the family Saccharomycetaceae. More than 100 species of this genus are known. The most well-known species include *Pichia anomala, Pichia guilliermondii, Pichia norvegensis,* and *Pichia ohmeri.*

*Pichia anomala* (formerly named *Hansenula anomala*) can be found in raw milk and cheese. The extracts of yeasts of the genus *Pichia* are rich in mannans, polysaccharides composed of mannose monomers. *Pichia anomala* and mannans are known to be used in the treatment of aging skin. See, for example, FR 2938768, FR 2906719, FR 2897266 and FR 2976490.

PRO-LIPISKIN® is a commercially available cosmetic ingredient containing extract of *Pichia anomala.* It is produced by a *Pichia* strain isolated from sugar cane. It is available from Silab-France.

US 2017/0172913A1 relates to topical compositions comprising combinations of *Pichia anomala* extract and chicory root extract that provide increased production of hyaluronic acid, along with methods of treating signs of skin aging and improving skin barrier protection and skin moisturization.

Soy products are well known skin care actives. Traditional Chinese medicine has used soy for thousands of years to reduce the appearance of skin discoloration. Genestein, an isoflavone found in soy, is known to stimulate hyaluronic acid production in transformed human keratinocytes. Miyazaki K, Hanamizu T, lizuka R, Chiba K. Genistein and daidzein stimulate hyaluronic acid production in transformed human keratinocyte culture and hairless mouse skin. Skin Pharmacol Appl Skin Physiol. 2002 May-Jun;15(3):175-83.

US Patent No. 7,192,615 relates to legume products having trypsin inhibitory activity and reduced microbial content, topical compositions containing them, and methods of decontaminating such products. AVEENO POSITIVELY RADIANT Body Lotion, commercially available from Johnson & Johnson Consumer Inc., contains glycine soja (soybean) seed extract.

Although the art provides topical uses for extracts of *Pichia anomala* and soy, applicants have now discovered that topical application of a combination of these two ingredients synergistically enhances the skin's production of hyaluronic acid from within. This unexpectedly provides significant benefits for skin, including improving, reducing, inhibiting, or delaying the appearance of at least one sign of aging in skin, and enhancing skin barrier protection and skin moisturization. Accordingly, new methods of treating signs of skin aging, for example, are now available.

### SUMMARY OF THE INVENTION

The present invention relates to a topical composition comprising an extract of *Pichia anomala* and a soy product.

The invention also relates to a method of treating a sign of skin aging, comprising topically applying to skin in need of treatment for skin aging a topical composition comprising an extract of *Pichia anomala* and a soy product.

The invention further provides a method of improving skin barrier function and moisturization, comprising topically applying to skin in need of improving skin barrier function and moisturization a topical composition comprising an extract of *Pichia anomala* and a soy product.

### DETAILED DESCRIPTION

The topical composition of the present invention improves the production of hyaluronic acid in the skin by synergistic action of extracts of *Pichia anomala* and soy products.

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, percentages used to express amounts of ingredients are percentage by weight (i.e., % (W/W). Similarly, weight ratios used to express relative proportions of ingredients are also determined using percentage by weight (i.e., weight ratios are calculated by dividing the percentage by weight of one ingredient by another). Unless stated otherwise, all ranges are inclusive of the endpoints, e.g., "from 4 to 9" includes the endpoints 4 and 9.

As used herein, a "product" is optionally in finished packaged form. The package may be a container, such as a plastic, metal or glass tube or jar containing the composition. The product may further contain additional packaging such as a plastic or cardboard box for storing such container. The product comprises a composition of the invention and may contain instructions directing the user to apply the composition to the skin or hair.

As used herein, "topically applying" means directly laying on or spreading on outer skin, the scalp, or hair, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically acceptable" means that the ingredients the term describes are suitable for use in contact with tissues (e.g., the skin or hair) without undue toxicity, incompatibility, instability, irritation, allergic response, or the like.

The compositions of the present invention may be suitable for treating signs of skin aging. As used herein, "signs of skin aging" includes the presence of lines and wrinkles, loss of elasticity, uneven skin, and blotchiness. Particularly preferably, the sign of aging is the presence of lines and wrinkles and/or loss of elasticity.

As used herein, "treating signs of skin aging" refers to mitigating, reducing, preventing, improving, or eliminating the presence or signs of skin aging described above.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

As used herein, "improving the firmness of skin" means the enhancing of the firmness or elasticity of the skin, preventing the loss of firmness or elasticity of skin, or preventing or treating sagging, lax and loose skin. The firmness or elasticity of the skin can be measured by use of a cutometer. See Handbook Of Non-Invasive Methods And The Skin, eds. J. Serup, G. Jemec & G. Grove, Chapter 66.1 (2006). The loss of skin elasticity or firmness may be a result of a number of factors, including but not limited to aging, environmental damage, or the result of an application of a cosmetic to the skin.

As used herein, "improving the texture of skin" means the smoothing of the surface of the skin to remove either bumps or crevasses on the skin surface.

As used herein, "improving the appearance of wrinkles in skin" means preventing, retarding, arresting, or reversing the process of wrinkle and fine line formation in skin.

As used herein, the term "safe and effective amount" means an amount sufficient to induce the desired effect, but low enough to avoid serious side effects. The safe and effective amount of the compound, extract, or composition will vary with, e.g., the age, health and environmental exposure of the end user, the duration and nature of the treatment, the specific extract, ingredient, or composition employed, the particular carrier utilized, and like factors.

As used herein, "skin in need of improving skin barrier function and moisturization" means skin that is, but not limited to, lacking in moisture, lacking in sebum, cracked, dry, itchy, scaly, xerodermic, dehydrated, lacks suppleness, lacks radiance, dull, or lacks lipids.

As described herein, applicants have discovered that topical application of a combination of an extract of *Pichia anomala* and a soy product provides unexpectedly good skin barrier function, skin moisturization, and skin anti-aging benefits.

Applicants have also discovered in particular that topical application of a composition containing a combination of an extract of *Pichia anomala* and a soy product enhances the endogenous hyaluronic acid ("HA") levels in skin, providing improvements in hydration and the appearance of at least one sign of skin aging. Topical use of such a composition can increase the levels of hyaluronic acid to a direction found in younger skin thereby providing the structural support to skin to reduce the appearance of signs of aging in skin.

### Pichia anomala

The topical composition comprises one or more extracts of *Pichia anomala.* In particular, such extracts may be extracts produced using one of the various strains of *Pichia anomala* isolated from the fruit or other aerial parts of a plant. Any cosmetically acceptable extract of *Pichia anomala* may be used.

One example of a suitable extract of *Pichia anomala* is PRO-LIPISKIN, commercially available from Silab-France. It is produced from a strain of *Pichia anomala* present on sugar cane.

Another example of a suitable extract of *Pichia anomala* is produced from a strain of *Pichia anomala* present on fruit or leaves of Kiwi plant.

The extract of *Pichia anomala* may be provided as a solution containing dry matter (the extract) in the range of about 20 wt%, more specifically 2 to 10 wt%, most specifically 3 to 7 wt%.

Solvents for such solutions include water, alcohols, glycols and the like. The solvent may be at least about 90 wt% water, or at least about 95 wt% water.

### Soy product

The topical composition also contains a soy product.

As used herein, "soy product" means a substance derived from the soybean.

The soy product may be soymilk or soymilk powder. Soymilk is a combination of solids derived from soybeans and water, the mixture of which has some or all the insoluble constituents filtered off. Soymilk powder is evaporated soymilk, which may be in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then be filtered to remove any insoluble residue. Second, soymilk may also be prepared from soybean powder. Soybean powder is thoroughly mixed with water (e.g., for at least one hour), which may then be followed by a filtration process to remove insoluble residues. Third, soymilk can also be reconstituted from soymilk powder by adding water. Alternatively or in addition, soymilk comprises from between about 1% to about 50%, by weight (e.g., from about 5% to about 20%, by weight) of solids from the soybean.

The surfaces of legume fruits such as soybeans often contain high levels of microorganisms. Thus, preferably the soy product may be treated to reduce or eliminate such microorganisms.

The soy product may have a total microbial content of less than about 10,000 colony-forming units ("cfu") per gram. The soy product may have a microbial content of less than about 1,000 cfu per gram (such as less than about 100 cfu per gram) of the soy product.

Alternatively or in addition, the soy product has a total objectionable microbial content of less than 300 cfu per gram such as less than 150 cfu per gram. The soy product may have an undetectable amount of any objectionable microbials for at least one gram (e.g., at least ten grams) of legume product.

Alternatively or in addition, the soy product is exposed to gamma irradiation. The soy product may be exposed to between about 2 to about 30 kGy of gamma irradiation, such as between about 5 and about 10 kGy of gamma irradiation. Such treatment reduces the microbial content of the soy product, while maintaining its biological activity. Applicants have also found that treatment of soy product with gamma irradiation maintains the cosmetic elegance of the soy product, such as its natural color and pleasant odor.

Other anti-microbial processes that maintain the protease inhibitory activity of the soy product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity, and combinations thereof. A complete list of methods for microbial content reduction is set forth in Disinfection, sterilization, and preservation 4th edition, Seymour S. Block, pp. 887-888 (1991, Lea & Febiger, Malvern, Pa.).

In certain cases, thermal treatment of the soy product may result in a substantial loss in protease inhibitory activity and, thus should be used with caution. For example, heating soymilk to 100° C for only 10 minutes may reduce the trypsin inhibitory activity of the soymilk from 86% (when maintained at 4° C.) to 46%. Heating soymilk can also result in a change of the color or odor of the soybean product.

### Amounts

Any suitable amount of *Pichia anomala* extract and soy product may be used in the compositions of the present invention. Preferably, the compositions comprise safe and effective amounts of both ingredients. In particular, the amounts of *Pichia anomala* extract and soy product used are cosmetically acceptable and are selected to achieve the desired treatment of skin for a particular condition, such as signs of aging, decreased barrier function, or decreased moisturization.

Preferably, the compositions may comprise from about 0.01 to about 1% by weight of *Pichia anomala* extract, more preferably about 0.026 to about 0.13% by weight of *Pichia anomala* extract.

Alternatively or in addition, preferably the compositions may comprise from about 0.1 to about 5, more preferably about 0.5 to about 2 of soy product.

Alternatively or in addition, the weight ratio of the extract of *Pichia anomala* to the soy product in the topical composition is about 1 to about 5, or about 2.5.

### Topical Compositions

The compositions of the present invention are applied topically to human skin or hair. Accordingly, the composition may further include a cosmetically acceptable topical carrier that may be from about 50% to about 99.99%, by weight, of the composition (e.g., from about 80% to about 99%, by weight, of the composition). Preferably, the cosmetically acceptable topical carrier may include water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Preferably, such compositions contain from about 2% to about 50% of an emollient(s). As used herein, "emollients" refer to materials used for the prevention or relief of dryness, such as by preventing the transepidermal loss of water from the skin. Examples of emollients include those known in the art. Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition of the present invention may include water or alternatively be anhydrous or be an ointment that includes no water but organic and/or silicone solvents, oils, lipids and waxes. An ointment may contain a simple base of animal or vegetable oils or semisolid hydrocarbons. An ointment may contain from about 2% to about 10% of an emollient(s) plus from about 0.1% to about 2% of a thickening agent(s).

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers are well known in the art.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The composition may be a gel cream. The gel cream aesthetic is characterized with a watery break, semi-translucent aspect and light after-feel. As used herein, the term "gel cream" means a formulation with low levels of oil droplets suspended in aqueous gel matrix.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

### Additional Cosmetically Active Agents

The compositions of the present invention may further comprise any of a variety of additional cosmetically active agents. Examples of suitable additional active agents include: skin lightening agents, darkening agents, additional anti-aging agents, tropoelastin promoters, collagen promoters, anti-acne agents, shine control agents, anti-microbial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH-changing agents, and the like.

Examples of various suitable additional cosmetically acceptable actives include hydroxy acids; benzoyl peroxide; D-panthenol; UV filters such as but not limited to avobenzone (PARSOL 1789), bisdisulizole disodium (NEO HELIOPAN AP), diethylamino hydroxybenzoyl hexyl benzoate (UVINUL A Plus), ecamsule (MEXORYL SX), methyl anthranilate, 4-aminobenzoic acid (PABA), cinoxate, ethylhexyl triazone (UVINULT 150), homosalate, 4-methylbenzylidene camphor (PARSOL 5000), octyl methoxycinnamate (Octinoxate), octyl salicylate (Octisalate), padimate O (ESCALOL 507), phenylbenzimidazole sulfonic acid (ENSULIZOLE), polysilicone-15 (PARSOL SLX), trolamine salicylate, Bemotrizinol (TINOSORB S), benzophenones 1-12, dioxybenzone, drometrizole trisiloxane (MEXORYL XL), iscotrizinol (UVASORB HEB), octocrylene, oxybenzone (EUSOLEX 4360), sulisobenzone, bisoctrizole (TINOSORB M), titanium dioxide, zinc oxide; carotenoids; free radical scavengers; spin traps; retinoids and retinoid precursors such as retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Shiitake mushroom extracts, and derivatives and mixtures thereof.

Preferably, the compositions may comprise a combination of *Pichia anomala* extract and soy products and at least one additional skin moisturizing active agent.

Alternatively or in addition, preferably the skin care compositions comprise a combination of *Pichia anomala* extract and soy product and at least one additional agent for improving the appearance of at least one sign of aging in skin. Examples of suitable additional agents improving the appearance of at least one sign of aging in skin include, but are not limited to, tropoelastin promoters, collagen promoters, retinoids, hyaluronic acid including cross-linked hyaluronic acid, dimethylaminoethanol, N,N,N',N'-tetrakis(2-hydroxypropyl)ethylenediamine, alpha hydroxy acids, polyhydroxyacids, sugar amines, and combinations of two or more thereof.

"Tropoelastin promoters," as used herein, refers to a class of compounds that possess the biological activity of enhancing the production of tropoelastin. Tropoelastin promoters, according to the present invention, include all natural or synthetic compounds that are capable of enhancing the production of tropoelastin in the human body.

Examples of suitable tropoelastin promoters include, but are not limited to, blackberry extracts, cotinus extracts, feverfew extracts, and bimetal complexes having copper and/or zinc constituents. The bimetal complex having copper and/or zinc constituents may be, for example, copper-zinc citrate, copper-zinc oxalate, copper-zinc tartarate, copper-zinc malate, copper-zinc succinate, copper-zinc malonate, copper-zinc maleate, copper-zinc aspartate, copper-zinc glutamate, copper-zinc glutarate, copper-zinc fumarate, copper-zinc glucarate, copper-zinc polyacrylic acid, copper-zinc adipate, copper-zinc pimelate, copper-zinc suberate, copper-zinc azealate, copper-zinc sebacate, copper-zinc dodecanoate, or combinations thereof. Preferably, the tropoelastin promoter may be selected from blackberry extracts, cotinus extracts, feverfew extracts, and combinations thereof. Particularly preferably, the tropoelastin promoter may be selected from blackberry extracts, feverfew extracts, and combinations thereof.

By "blackberry extract," it is meant a blend of compounds isolated from the plant of the genus *Rubus,* and preferably *Rubus fruticosus.* The compounds may be isolated from the flowers of the plant. The compounds may be isolated from dried flowers of the plant. Such compounds may be isolated from one or more part of the plant (e.g., the whole plant, flower, seed, root, rhizome, stem, fruit and/or leaf of the plant). Preferably, the blackberry extract may be a blackberry leaf extract. One particularly suitable blackberry extract is produced by extracting the leaves of *Rubus fruticosus* with a mixture of water and ethanol compounded to an activity of about 5% to about 10%, with a maltodextrin matrix, commercially available from Symrise Inc. of Teterboro, NJ, and is sold under the name SYMMATRIX.

Compositions of the present invention may include a cosmetically effective amount of one or more tropoelastin promoters such as those described above. The compositions preferably include, on an active basis, from about 0.1% to about 10% of the tropoelastin promoters, more preferably from about 0.5% to about 5% of tropoelastin promoters, and most preferably from about 0.5% to about 2% of the tropoelastin promoters.

"Collagen promoter," as used herein, refers to compounds that possess the biological activity of enhancing the production of collagen. "Non-retinoid collagen promoters" according to the present invention include all natural or synthetic compounds that are not retinoids, or derived from retinoids, and are capable of enhancing the production of collagen in the human body.

Examples of suitable collagen promoters include, but are not limited to the following: Retinoids including retinol, retinaldehyde, and retinoic acid, extracts of feverfew (*Tanacetum parthenium*), extracts of *Centella asiatica,* and extracts of *Siegesbeckia orientalis*; collagen-promoting peptides; ursolic acid; and asiaticoside.

*Centella asiatica,* also known as *Violette marronne* on Reunion Island, Gotu Kola or Indian pennywort in India, *Centella repanda* in North America, and Talapetraka in Madagascar, is a polymorphous herb and belongs to the family of *Umbelliferae* (*Apiaceae*), particularly to the *Hydrocotyle* subfamily. It grows wild throughout the tropics and prefers moist and shady regions at an altitude of about 600 to 1200 meters above sea level. *Centella asiatica* has three varieties: Typica, Abyssinica, and Floridana. The herb is known and used for its healing, sedative, analgesic, antidepressant, antiviral and antimicrobial properties. The biological activity of the herb appears to be due to the presence of triterpene molecules in the herb. A suitable extract of *Centella asiatica* is available as TECA from Bayer Consumer HealthCare of Basel, Switzerland.

By "extracts of *Siegesbeckia orientalis,"* is meant any of various extracts of the plant *Siegesbeckia orientalis,* including Darutoside available from Sederma (Croda International Group of Edison, NJ).

Suitable collagen-promoting peptides include the following matrikine peptides, (*i.e.*, a peptide derived from the degradation of extracellular matrix proteins - collagen, elastin, or proteoglycan) including palmitoyl pentapeptides, such as MATRIXYL from Sederma (Croda International Group of Edison, NJ); GHK copper peptide available as PROCYTE from Photomedex of Montgomeryville, PA; Palmitoyl GHK peptide available as Biopoeptide CL from Sederma (Croda International Group of Edison, NJ); Biomimetic tetrapeptides, such as those available as Chronoline Tri Peptide from Unipex of Québec, Canada; and Palmitoyl tripeptide, available as Syn-Coll from DSM of Basel, Switzerland.

Ursolic acid is also known as pentacyclic triterpene acid, Prunol, Malol, Urson, beta-ursolic acid and 3-Beta-Hydroxy-Urs-12-En-28-Oic Acid. It is commercially available for example from Sigma-Aldrich of St. Louis, MO.

Asiaticoside, also known chemically as: [6-[[3,4-dihydroxy-6-(hydroxymethyl)-5-(3,4,5-trihydroxy-6-methyloxan-2-yl)oxyoxan-2-yl]oxymethyl]-3,4,5-trihydroxyoxan-2-yl] 10,11-dihydroxy-9-(hydroxymethyl)-1,2,6a,6b,9,12a-hexamethyl-2,3,4,5,6,6a,7,8,8a,10,11,12,13,14b-tetradecahydro-1H-picene-4a-carboxylate) is commercially available for example from Bayer Santé Familiale Division Serdex, 69, Boulevard Victor Hugo 93400 SAINT-OUEN France.

Compositions of the present invention may include a cosmetically effective amount of one or more collagen promoters. Preferably, the compositions include, on an active basis, from about 0.1% to about 10% of the collagen promoters, more preferably from about 0.5% to about 5% of collagen promoters, and most preferably from about 0.5% to about 2% of the collagen promoters.

The compositions of the present invention may comprise additionally at least one skin lightening active agent. Examples of suitable skin lightening active agents include, but are not limited to, tyrosinase inhibitors, melanin-degradation agents, melanosome transfer inhibiting agents including PAR-2 antagonists, exfoliants, sunscreens, retinoids, antioxidants, Tranexamic acid, tranexamic acid cetyl ester hydrochloride, skin bleaching agents, linoleic acid, adenosine monophosphate disodium salt, Chamomilla extract, allantoin, opacifiers, talcs and silicas, zinc salts, and the like, and other agents as described in Solano et al. Pigment Cell Res. 19 (550-571) and Ando et al. Int J Mol Sci 11 (2566-2575).

Examples of suitable tyrosinase inhibitors include, but are not limited to, Vitamin C and its derivatives, Vitamin E and its derivatives, Kojic Acid, Arbutin, resorcinols, hydroquinone, Flavones e.g. Licorice flavanoids, Licorice root extract, Mulberry root extract, Dioscorea Coposita root extract, Saxifraga extract and the like, Ellagic acid, Salicylates and derivatives, Glucosamine and derivatives, Fullerene, Hinokitiol, Dioic acid, Acetyl glucosamine, 5,5'-dipropyl-biphenyl-2,2'-diol (Magnolignan), 4-(4-hydroxyphenyl)-2-butanol (4-HPB), combinations of two or more thereof, and the like. Examples of vitamin C derivatives include, but are not limited to, ascorbic acid and salts, Ascorbic Acid-2-Glucoside, sodium ascorbyl phosphate, magnesium ascorbyl phosphate, and natural extract enriched in vitamin C. Examples of vitamin E derivatives include, but are not limited to, alpha-tocopherol, beta-tocopherol, gamma-tocopherol, delta-tocopherol, alpha-tocotrienol, beta-tocotrienol, gamma-tocotrienol, delta-tocotrienol and mixtures thereof, tocopherol acetate, tocopherol phosphate and natural extracts enriched in vitamin E derivatives. Examples of resorcinol derivatives include, but are not limited to, resorcinol, 4-substituted resorcinols like 4-alkylresorcinols such as 4-butyresorcinol (rucinol), 4-hexylresorcinol (Synovea HR, Sytheon), phenylethyl resorcinol (Symwhite, Symrise), 1-(2,4-dihydroxyphenyl)-3-(2,4-dimethoxy-3-methylphenyl)-Propane (nivitol, Unigen) and the like and natural extracts enriched in resorcinols. Examples of salicylates include, but are not limited to, 4-methoxy potassium salicylate, salicylic acid, acetylsalicylic acid, 4-methoxysalicylic acid and their salts. Preferably, the tyrosinase inhibitors may include a 4-substituted resorcinol, a vitamin C derivative, or a vitamin E derivative. More preferably, the tyrosinase inhibitor comprise Phenylethyl resorcinol, 4-hexyl resorcinol, or ascorbyl-2-glucoside.

Examples of suitable melanin-degradation agents include, but are not limited to, peroxides and enzymes such as peroxidases and ligninases. Preferably, the melanin-inhibiting agents may include a peroxide or a ligninase.

Examples of suitable melanosome transfer inhibiting agents including PAR-2 antagonists such as Vitamin B3 and derivatives such as Niacinamide.

Examples of exfoliants include, but are not limited to, alpha-hydroxy acids such as lactic acid, glycolic acid, malic acid, tartaric acid, citric acid, or any combination of any of the foregoing, beta-hydroxy acids such as salicylic acid, polyhydroxy acids such as lactobionic acid and gluconic acid, and mechanical exfoliation such as microdermabrasion. Preferably, the exfoliants may include glycolic acid or salicylic acid.

Examples of retinoids include, but are not limited to, retinol (Vitamin A alcohol), retinal (Vitamin A aldehyde), retinyl acetate, retinyl propionate, retinyl linoleate, retinoic acid, retinyl palmitate, isotretinoin, tazarotene, bexarotene, Adapalene, combinations of two or more thereof and the like. Preferably, the retinoid may be selected from the group consisting of retinol, retinal, retinyl acetate, retinyl propionate, retinyl linoleate, and combinations of two or more thereof. More preferably, the retinoid may be retinol.

Examples of antioxidants include, but are not limited to, water-soluble antioxidants such as sulfhydryl compounds and their derivatives (*e.g*., sodium metabisulfite and N-acetylcysteine, glutathione), lipoic acid and dihydrolipoic acid, stilbenoids such as resveratrol and derivatives, lactoferrin, iron and copper chelators and ascorbic acid and ascorbic acid derivatives (*e.g*., ascobyl-2-glucoside, ascorbyl palmitate and ascorbyl polypeptide). Oil-soluble antioxidants suitable for use in the compositions of this invention include, but are not limited to, butylated hydroxytoluene, retinoids (*e.g*., retinol and retinyl palmitate), tocopherols (*e.g*., tocopherol acetate), tocotrienols, and ubiquinones. Natural extracts containing antioxidants suitable for use in the compositions of this invention, include, but not limited to, extracts containing flavonoids and isoflavonoids and their derivatives (*e.g*., genistein and diadzein), extracts containing resveratrol and the like. Examples of such natural extracts include grape seed, green tea, black tea, white tea, pine bark, feverfew, parthenolide-free feverfew, oat extracts, blackberry extract, cotinus extract, pomelo extract, wheat germ extract, Hesperedin, Grape extract, Portulaca extract, Licochalcone, chalcone, 2,2'-dihydroxy chalcone, Primula extract, propolis, and the like.

The additional cosmetically active agent may be present in a composition in any suitable amount, for example, in an amount of from about 0.0001% to about 20% by weight of the composition, e.g., about 0.001% to about 10% such as about 0.01% to about 5%. Preferably, the additional cosmetically active agent may be present in an amount of 0.1% to 5% and preferably may be present in an amount from 1% to 2%.

Compositions of the present invention may include a cosmetically effective amount of one or more anti-inflammatory compounds.

Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Feverfew (*Tanacetum parthenium*), Ginger (*Zingiber officinale*), Ginko (*Ginkgo biloba*), Madecassoside (*Centella asiatica* extract ingredient), Cotinus (*Cotinus coggygria*), Butterbur Extract (*Petasites hybridus*), Goji Berry (*Lycium barbarum*), Milk Thistle Extract (*Silybum marianum*), Honeysuckle (*Lonicera japonica*), Basalm of Peru (*Myroxylon pereirae*), Sage (*Salvia officinalis*), Cranberry Extract (*Vaccinium oxycoccos*), Amaranth Oil (*Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*), Licochalcone (Licorice: *Glycyrrhiza inflate* extract ingredient), Symrelief (Bisabolol and Ginger extract), combinations of two or more thereof, and the like.

The anti-inflammatory agent may be a resorcinol. Particularly suitable substituted resorcinols include 4-hexyl resorcinol and 4-octylresorcinol, particularly 4-hexyl resorcinol. 4-Hexyl resorcinol is commercially available as SYNOVEA HR from Sytheon of Lincoln Park, NJ. 4-Octylresorcinol is commercially available from City Chemical LLC of West Haven, Connecticut.

By "extracts of feverfew," it is meant extracts of the plant *"Tanacetum parthenium,"* such as may be produced according to the details set for the in US Patent Application Publication No. 2007/0196523, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

In the skin care composition of the invention, the ratio of the amounts of the combined *Pichia anomala* extract and soy products to the anti-inflammatory compound may be varied. For example, the extract and the anti-inflammatory compound may be present in a weight ratio (which is determined by dividing the amount by weight of the dry extract by the amount by weight of the anti-inflammatory compound) of about 0.001 to about 100, preferably about 0.01 to about 10, more preferably about 0.25 to about 2.

A variety of other materials may also be present in the compositions of the present invention. Preferably, the composition may comprise one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (e.g., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (*e.g*., hygroscopic compounds). Examples of suitable humectants include those found Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (*e.g*., α,α-trehalose, β,β-trehalose, α,β-trehalose) or a salt or ester thereof (*e.g*., trehalose 6-phosphate).

The composition may contain glycerin. For example, the composition contains at least 5 wt % glycerin. The composition may contain at least 8 wt % glycerin.

Alternatively or in addition, the composition has a pH of 6.5 or less. For example, the composition may have a pH of 5.5 or less.

Alternatively or in addition, the composition contains about 8 wt% glycerin and has a pH or 6.5 or less. Such a composition provides substantial increases in hyaluronic acid production when used to treat skin according to the invention.

Alternatively or in addition, the composition comprises 0.1 to 5 weight percent of cetearyl olivate. The composition may comprise 0.1 to 3 weight percent of cetearyl olivate.

Alternatively or in addition, the composition comprises 0.1 to 5 weight percent of sorbitan olivate. The composition may comprise 0.1 to 3 weight percent of sorbitan olivate.

A convenient source of cetearyl olivate and sorbitan olivate is Olivem 1000, commercially available from Hallstar Itali.

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc New York, NY) and include, but are not limited to anionic surfactants such as sulfates, cationic surfactants such as betaines, amphoteric surfactants such as sodium coco glycinate, noionic surfactants such as alkyl polyglucosides.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the trade name VERSENE 100XL.

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and preferably include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)ₐ-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Any fragrance compositions suitable for use on skin may be used in accord with the present invention.

Preferably, the present invention may be in the form of a substrate comprising a composition of the present invention. Any suitable substrate may be used. Examples of suitable substrates and substrate materials are disclosed, for example, in U.S. Published Application Nos. 2005/0226834 and 2009/0241242 which are incorporated herein by reference in their entirety.

Preferably, the substrate may be a wipe, glove, or a facial mask, preferably comprising a water-insoluble substrate such as is defined in the cited references above. The water-insoluble substrate may have a size and shape such that it covers the face of a human user to facilitate placing the water-insoluble substrate about the face of the user as a mask substrate. For example, the water-insoluble mask substrate may have openings for a mouth, nose, and/or eyes of the user. Alternatively, the water-insoluble substrate may have no such openings. Such a configuration without openings may be useful when the water-insoluble substrate is intended to be draped over a non-facial expanse of skin or if the water-insoluble substrate is intended to be used as wipe. The water-insoluble substrate may have various shapes, such as an angular shape (*e.g*., rectangular) or an arcuate shape such as circular or oval. The substrate may be a glove such as described in U.S. Published Application No 2006/0141014 which is incorporated herein in its entirety. The product may include a plurality of water-insoluble substrates of different shapes.

The present invention further comprises a method of improving the barrier function and moisturization of skin by applying to skin in need of improving skin barrier function and moisturization a combination of *Pichia anomala* extract and soy product. The method comprises for example topically applying a composition of the present invention comprising *Pichia anomala* extract and soy product to skin in need of improving skin barrier function and moisturization. Such topical application may be to any skin in need of treatment on the body, for example skin of the face, lips, neck, chest, back, arms, axilla, hands, feet and/or legs. Preferably, the combined *Pichia anomala* extract and soy product are applied in an effective amount that results in the desired improvement of skin barrier function being achieved.

The present invention further comprises a method of improving the appearance of at least one sign of skin aging by applying to skin in need of improving the appearance of at least one sign of skin aging a combination of *Pichia anomala* extract and soy product. The method comprises for example topically applying a composition of the present invention comprising *Pichia anomala* extract and soy product to skin in need of treatment of at least one sign of skin aging. Such topical application may be to any skin in need of treatment on the body, for example skin of the face, lips, neck, chest, back, arms, axilla, hands, feet and/or legs. Preferably, the *Pichia anomala* extract and soy product are applied in an effective amount that results in the desired improvement in the appearance of at least one sign of skin aging being achieved.

Any suitable method of applying the composition to the skin in need may be used. For example, the composition may be applied directly from a package to the skin in need, by hand to the skin in need, or may be transferred from a substrate such as a wipe or mask, or a combination of two or more thereof. The composition may be applied via a dropper, tube, roller, spray, and patch or added to a bath or otherwise to water to be applied to the skin, and the like. The composition may be applied in a variety of manners /forms, including, without limitation, as a leave-on cream, mask, and / or serum.

The methods of the present invention may comprise applying at least two different compositions or products comprising *Pichia anomala* extract or soy product to the skin. For example, the methods may comprise applying a first composition comprising an extract of *Pichia anomala,* followed by applying a second composition comprising a soy product that is different from the first composition, to the skin in need of treatment.

Preferably, the first and second composition may be independently selected from the group consisting of lotions, cleansers, masks, wipes, creams, serums, gels, and the like. Preferably, at least one of the first and second compositions may be a cleanser, lotion, cream, essence, or serum and the other is a facial mask or wipe. Alternatively or in addition, preferably at least one of the first and second compositions is a cleanser and the other is a lotion or cream.

The composition and formulations and products containing such compositions of the present invention may be prepared using methodology that is well known by an artisan of ordinary skill. These compositions may be useful in treating skins of aging such as wrinkles, loss of elasticity, uneven skin including reducing blotchiness. The composition may be used on a routine basis and is substantially free of skin irritants.

The following non-limiting examples further illustrate the present invention.

### Example 1

Hyaluronic acid (HA) production in human dermal fibroblasts after treating the cells with an extract of *Pichia anomala* and/or soy product were determined and compared as follows.

The soy product was Glycine Soja Seed Extract (commercially available from Devansoy, Inc., Rock City, IL) and was a powder. It was re-suspended in Phosphate Buffer Saline (PBS) to make a 10% (weight/volume) stock solution. This stock solution was then further diluted in the cell culture medium to reach the final tested concentrations.

The extract of *Pichia anomala* was in the form of a 5% aqueous solution. The *Pichia anomala* had been grown on kiwi plant. The 5% aqueous solution was treated as a 100% stock solution.

The two ingredients were added to the culture media either alone or together at the described doses and their resulting effects on HA production were used to determine fold change of HA production compared with untreated.

The human dermal fibroblasts were maintained in flask in growth medium consisting of Dulbeccos' Modified Eagle Medium (DMEM) plus 10% fetal bovine serum, 50 units/ml penicillin and 50µg/ml streptomycin. Cells were seeded at 20,000 cells per well in a 96 well plate. After 24 hours incubation, the cells were treated with the test compositions dissolved in DMSO or DMSO alone (as the control) prepared in DMEM+2%FBS. Culture media was collected at 48 hours post-treatment, and measured for HA secretion using Hyaluronan ELISA kit (Echelon, cat. #K-1200) following the manufacturer's protocol. To assess activity, the colorimetric chance was measured at 405 nm.

The results are shown in Table 1. The results are expressed as percent increase in HA production over untreated.

**TABLE 1**

| | **Wt % Soy Product** | | | | |
|---|---|---|---|---|---|
| **Wt % *Pichia anomala* Extract Solution (wt% *Pichia anomala* Extract)** | | 0 | 0.01 | 0.025 | 0.05 |
| | 0 | na | 16.39 | 39.02 | 73.61 |
| | 0.5 (0.013) | 27.34 | 80.55 | 97.06 | 138.91 |
| | 1 (0.026) | 66.08 | 115.40 | 142.59 | 195.92 |

The results in Table 1 show treatment of skin cells with combinations of *Pichia anomala* extract and soy product resulted in unexpectedly high and synergistic increases HA production by the cells. All the combinations provided increases in HA production greater than the expected additive increases from the ingredients separately.

For example, 0.5 wt% *Pichia anomala* extract solution provided a 27.34% increase in HA production versus untreated, and 0.01 wt% soy product provided a 16.39% increase in HA production versus untreated. The expected additive increase by combining the two was 43.73%. However, the actual increase versus untreated was 80.55%, almost double the expected additive increase.

### Example 2

Hyaluronic acid (HA) production by human skin explants was determined and compared after treating them with test Compositions A, B and C (comparative) and Compositions 1 and 2 (according to the invention) containing different amounts of *Pichia anomala* extract and soy product. The test compositions contained the ingredients shown in Table 2.

**TABLE 2**

| **US INCI Name** | **Placebo (w/w %)** | **Composition A (w/w %)** | **Composition B (w/w %)** | **Composition 1 (w/w %)** | **Composition C (w/w %)** | **Composition 2 (w/w %)** |
|---|---|---|---|---|---|---|
| Water | 0.18 | 0.18 | 0.18 | 0.18 | 0.18 | 0.18 |
| Sodium Hydroxide | 0.02 | 0.02 | 0.02 | 0.02 | 0.02 | 0.02 |
| Dimethicone; Dimethicone Crosspolymer | 1.50 | 1.50 | 1.50 | 1.50 | 1.50 | 1.50 |
| Dimethicone | 3.50 | 3.50 | 3.50 | 3.50 | 3.50 | 3.50 |
| Dimethicone; Dimethiconol | 2.00 | 2.00 | 2.00 | 2.00 | 2.00 | 2.00 |
| Soy product | 0.00 | 0.00 | 2.00 | 2.00 | 0.00 | 2.00 |
| Mica; Titanium Dioxide | 0.50 | 0.50 | 0.50 | 0.50 | 0.50 | 0.50 |
| Water | 2.00 | 2.00 | 2.00 | 2.00 | 2.00 | 2.00 |
| Water | 75.43 | 70.43 | 73.43 | 72.43 | 72.93 | 70.93 |
| Carbomer | 0.15 | 0.15 | 0.15 | 0.15 | 0.15 | 0.15 |
| Synthetic Beeswax | 0.50 | 0.50 | 0.50 | 0.50 | 0.50 | 0.50 |
| Cetearyl Olivate; Sorbitan Olivate | 2.00 | 2.00 | 2.00 | 2.00 | 2.00 | 2.00 |
| Chlorphenesin | 0.27 | 0.27 | 0.27 | 0.27 | 0.27 | 0.27 |
| Polyacrylamide; Laureth-7; C13-14 Isoparaffin | 2.00 | 2.00 | 2.00 | 2.00 | 2.00 | 2.00 |
| Dimethicone/Vinyl Dimethicone Crosspolymer; C12-14 Pareth-12 | 1.00 | 1.00 | 1.00 | 1.00 | 1.00 | 1.00 |
| Actinidia Chinensis (Kiwi) Fruit Water | 0.10 | 0.10 | 0.10 | 0.10 | 0.10 | 0.10 |
| Fragrance | 0.25 | 0.25 | 0.25 | 0.25 | 0.25 | 0.25 |
| *Pichia anomala* Extract Solution (wt% *Pichia anomala* Extract) | 0.00 | 5.00 (0.13) | 0.00 | 2 (0.052) | 2 (0.052) | 5 (0.13) |
| Ethylhexylglycerin; Phenoxyethanol | 0.60 | 0.60 | 0.60 | 0.60 | 0.60 | 0.60 |
| Glycerin | 8.00 | 8.00 | 8.00 | 8.00 | 8.00 | 8.00 |

The *Pichia anomala* extract was prepared as described in Example 1.

The test compositions were prepared as follows.

A 10% sodium hydroxide solution was made by dissolving sodium hydroxide in water.

A Premix 2 was made in by first adding Cyclopentasiloxane (and) Dimethicone/Vinyldimethicone Crosspolymer to a separate vessel, and then adding Dimethicone; Dimethiconol, and mixing until uniform. Premix 2 was set aside until added to main vessel.

A Premix 3 was made in a separate vessel by adding Glycine Soja Seed Extract (commercially available from Devansoy, Inc., Rock City, IL) to glycerin, and mixing until uniform.

A Premix 4 was made in a separate premix vessel by adding the Mica; Titanium Dioxide in water, and mixing until uniform.

Next, water was added to a main vessel and mixing begun. Carbomer was slowly sprinkled into the main vessel and mixed until fully hydrated. Next, the main vessel was heated to 70-75° C. At 70-75° C, the batch was partially neutralized using the 10% sodium hydroxide solution (pH 5.0 - 6.0, target 5.5), and mixed until a clear, free flowing solution. The mixing speed was monitored to avoid aeration. While batch was still at 70-75° C, the chlorphenesin was added and mixed until fully dissolved. Next, the batch was heated to 80-85°C, and the beeswax and cetearyl olivate/sorbitan olivate added. The batch was then mixed for 15 minutes or until a smooth uniform bulk formed. The batch was cooled to 70° C and Polyacrylamide; Laureth-7; C13-14 Isoparaffin added and mixed until uniform. The batch was cooled to 40° C and Premix 2 was added and mixed until uniform. The Dimethicone/Vinyl Dimethicone Crosspolymer; C12-14 Pareth-12 was then added and mixed until uniform. Next, the phenoxyethanol was added and mixed until uniform. At 30-35° C, Premix 3, Premix 4, *Pichia anomala* extract solution and fragrance were added to the main batch, and mixed until uniform. The pH was maintained in the range of 5.0-6.0 (target 5.5). Adjustments to pH were made as necessary with the 10% sodium hydroxide solution. Finally, the composition was homogenized until smooth and uniform.

HA production by the compositions was determined using immunohistology on normal explants of human skin from three donors (29, 30, 55 years old). Eight mm diameter punches were cut from the explants and deposited on pieces of sterile gauze and placed, one explant per well, in six well plates with 3mL of culture media. The culture media was sold under the tradename GIBCO DMEM/F-12 (ThermoFisher Scientific, Waltham, MA, catalog #11514436) with 1% GIBCO Penicillin-Streptomycin (ThermoFisher Scientific, catalog #11528876) and 0.1% amphotericin B sold under the tradename FUNGIZONE (ThermoFisher Scientific, catalog #11510496).

For each test composition, 5 µl of test composition was applied to an explant once a day for 5 days. The placebo composition was used as the control. On day 7 the explants were recovered, wiped with a sterile gauze, then cut in half vertically and fixed in 4% paraformaldehyde (V/V). On day 8 the explants were dehydrated and embedded in paraffin. Each test composition was tested in triplicate.

The paraffinized slides were stripped with xylene and epitope retrieval was carried out with PT link (Agilent, Santa Clara, CA) and target retrieval solution sold under the tradename ENVISION Flex, High pH (Dako, DM828, Agilent, Santa Clara, CA). Slides were then rinsed with wash buffer sold under the tradename ENVISION (Dako, DM831, Agilent, Santa Clara, CA) one time for 10 mins. Permeabilization and saturation were done with PBS 0.3% Triton/5% goat serum (Dako, Santa Clara, CA, catalog #CP3418/X090710-8) for 30 mins, followed by labeling with Hyaluronic Acid Binding Protein ("HAPB" from Calbiochem, catalog #385911, Millipore Sigma, St. Louis, NJ) overnight at 4°C. The next day the slides were rinsed with PBS three times for 5 minutes each. Antibody was revealed with biotin-binding protein covalently attached to a fluorescent label sold under the tradename ALEXA FLUOR 488 streptavidin (Invitrogen™, catalog #S11223) and staining of nuclei was done with DAPI solution (Dako, Santa Clara, CA) at 5µg/ml for 30 min at ambient temperature. Slides were then rinsed with PBS and mounted with Fluoprep mounting medium (bioMerieux UK Ltd.,UK catalog #75521).

Pictures of the skin sections were taken with an Olympus IX70 Fluorescence microscope (Olympus Corporation, Japan) coupled to an image analysis system (NIS-Elements AR software, Nikon Instruments, Melville, NY). Quantitative analysis of images was conducted with ImageJ software.

The results are shown in Table 3. The results are expressed as average fluorescence intensity of the dermis (in Arbitrary Units (AU)). Fluorescence intensity (green) is proportional to the synthesis of HABP.

**TABLE 3**

| **Composition** | **Count** | **Sum** | **Average** | **Variance** | **% increase vs placebo** |
|---|---|---|---|---|---|
| Placebo | 6 | 47.713 | 7.952167 | 7.317858 | |
| Composition 2 | 6 | 93.16 | 15.52667 | 3.043953 | 95.3 |
| Composition C | 6 | 61.265 | 10.21083 | 8.078452 | 28.4 |
| Composition 1 | 6 | 68.525 | 11.42083 | 19.20708 | 43.6 |
| Composition A | 6 | 73.748 | 12.29133 | 1.772167 | 54.6 |
| Composition B | 6 | 55.838 | 9.306333 | 8.660897 | 17 |

Treatment of skin explants with Composition 2 containing a combination of 0.13 wt% *Pichia anomala* extract and 2% wt% soy product resulted in unexpectedly high and synergistic increases HA production versus placebo. This combination provided increases in HA production greater than the expected additive increases from the ingredients separately.

Specifically, Composition A containing 0.13 wt% *Pichia anomala* extract provided a 54.6% increase in HA production versus placebo, and Composition B containing 2 wt% soy product provided a 17% increase in HA production versus placebo. The expected additive increase by combining the two is 71.6%. However, the actual increase in HA production achieved with Composition 2 versus placebo was 95.3%, 23.7% more than the expected additive increase.

A non-exhaustive list of aspects of the invention are set out in the following clauses:

### Clauses of the invention:

1. A topical composition comprising an extract of *Pichia anomala* and a soy product.
2. The topical composition of clause 1, wherein the extract of *Pichia anomala* is prepared from a strain of *Pichia anomala* present on kiwi fruit or leaves.
3. The topical composition of clause 1, wherein the extract of *Pichia anomala* is prepared from a strain of *Pichia anomala* present on sugar cane.
4. The topical composition of clause 1, wherein the weight ratio of the extract of *Pichia anomala* to the soy product in the topical composition is about 1 to about 5.
5. The topical composition of clause 1 comprising about 0.01 to about 1 weight percent of the extract of *Pichia anomala.*
6. The topical composition of clause 1 comprising about 0.1 to about 5 weight percent of soy product.
7. The topical composition of clause 1 further comprising at least about 5 wt % glycerin.
8. The topical composition of clause 1 having a pH of about 6.5 or less.
9. A method of treating a sign of skin aging, comprising topically applying to skin in need of treatment for skin aging a topical composition comprising an extract of *Pichia anomala* and a soy product.
10. A method of improving skin barrier function and moisturization, comprising topically applying to skin in need of improving skin barrier function and moisturization a topical composition comprising an extract of *Pichia anomala* and a soy product.

## Claims

1. A topical composition comprising an extract of *Pichia anomala* and a soy product.

2. The topical composition of claim 1, wherein the extract of *Pichia anomala* is prepared from a strain of *Pichia anomala* present on kiwi fruit or leaves.

3. The topical composition of claim 1, wherein the extract of *Pichia anomala* is prepared from a strain of *Pichia anomala* present on sugar cane.

4. The topical composition of any preceding claim, wherein the weight ratio of the extract of *Pichia anomala* to the soy product in the topical composition is about 1 to about 5.

5. The topical composition of any preceding claim comprising about 0.01 to about 1 weight percent of the extract of *Pichia anomala.*

6. The topical composition of any preceding claim comprising about 0.1 to about 5 weight percent of soy product.

7. The topical composition of any preceding claim further comprising at least about 5 wt % glycerin.

8. The topical composition of any preceding claim having a pH of about 6.5 or less.

9. A method of treating a sign of skin aging, comprising topically applying to skin in need of treatment for skin aging a topical composition comprising an extract of *Pichia anomala* and a soy product.

10. A method of improving skin barrier function and moisturization, comprising topically applying to skin in need of improving skin barrier function and moisturization a topical composition comprising an extract of *Pichia anomala* and a soy product.
